(19) Europäisches Patentamt
European Patent Office
Office européen des brevets

(11) EP 2 210 585 A1

(12) **EUROPEAN PATENT APPLICATION**

(43) Date of publication:
28.07.2010 Bulletin 2010/30

(51) Int Cl.:
*A61K 9/00* (2006.01)  *A61K 9/20* (2006.01)
*A61K 31/567* (2006.01)

(21) Application number: 09290034.9

(22) Date of filing: 16.01.2009

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(71) Applicant: Exelgyn
75007 Paris (FR)

(72) Inventor: Prinderre, Pascal
13004 Marseille (FR)

(74) Representative: Hirsch & Associés
58, avenue Marceau
75008 Paris (FR)

(54) **SPRM pharmaceutical compositions methods of treatment using them**

(57) The invention aims at providing novel compositions comprising Selective Progesterone Receptor Modulator (SPRM) and novel methods of treatment using them, which have the ability to prevent the intake of the composition by the patient in an abortive amount and avoid their misuse.

Figure 4

EP 2 210 585 A1

Printed by Jouve, 75001 PARIS (FR)

**Description**

**FIELD OF THE INVENTION**

The invention relates to novel Selective Progesterone Receptor Modulator (SPRM) compositions and to methods of treatment using them, especially where the SPRM is mifepristone.

**BACKGROUND OF THE INVENTION**

Mifepristone, also known as RU486 has the chemical formula 11β-[p-(Dimethylamino)phenyl]-17β-hydroxy-17-(1-propynyl)estra-4,9-dien-3-one, and is an important antiprogestin that effectively and safely terminates early pregnancy, when used in conjunction with a prostaglandin. It also used for cervix dilation before subsequent termination of pregnancy during the first trimester by vacuum aspiration, preparation of the cervix for termination of pregnancy for medical reasons after the first trimester, and induction of labor when the fetus has died in the uterus.

Mifepristone is commercially available in a dose of 3 tablets of 200 mg under the trade name Mifegyne®. For the interruption of pregnancy a prostaglandin is administered 36 to 48 hours afterwards.

A novel therapeutical use of mifepristone has been recently discovered. It is disclosed in Murphy, A.A et al., "Regression of Uterine Leiomyomata in Response to the Antiprogesterone RU 486", (1993) Journal of Clinical Endocrinology and Metabolism vol. 76, No. 2, that mifepristone may be used to treat leiomyomata.

Leiomyomata are common pelvic fibroid tumors occurring in up to 20% of women over 30 years of age. Leiomyomata represent one of the most frequent indications of operative procedures in woman of reproductive age. Symptoms are reported in twenty to fifty percent of cases of leiomyomata and include pelvic pain, excessive duration or amount of menstruation, infertility and pelvic masses. The anti-progestin activity of mifepristone inhibits the growth of such tumors.

The dosage for this therapeutical treatment is however far different from the dosage for termination of pregnancy. Indeed, the dosage is about 25 mg per day, especially 2 to 20 mg per day and preferably 5 mg per day.

However, one should ensure that a woman that would be pregnant cannot take as many unitary dosage forms originally designed for the treatment of leiomyomata, and then reach the 200 mg amount.

Formulations to avoid the misuse of drugs are already disclosed in Canadian Patent Application CA 2,029,940, which relates to retentive delivery devices in the form of antioestrogen or antiprogestin containing injectable microspheres. These are injected under the skin, and release a constant amount of an antioestrogen or antiprogestin over a period of 1 to 3 months, avoiding by the same way any overdosage or the misuse of the drug by the patient. However, this Application does not disclose oral formulations, nor consider mifepristone as an antiprogestin within the scope of the invention.

There is a need of a new mifepristone or SPRM dosage form that would ensure that only a limited number of unitary dosage forms can be administered orally to a woman.

**SUMMARY OF THE INVENTION**

The invention aims at providing novel compositions comprising Selective Progesterone Receptor Modulator (SPRM) and novel methods of treatment using them, which have the ability to prevent the intake of the composition by the patient in an abortive amount and avoid their misuse.

The invention thus aims at providing compositions and methods which brings one or more of the following advantages:

- the compositions exhibit a swelling into the stomach, such that unpleasant feeling is obtained when a low amount, especially three, unitary dosage forms are taken by the patient.
- the compositions contain a laxative component in such an amount that the patient would feel uncomfortable as soon as the number of compositions taken reaches a low number, especially three compositions.
- generally speaking the composition contains an excipient of a nature and content such that this will prevent misuse (or misusage) of the mifepristone or SPRM active ingredient.

A first aspect of the invention is directed to a composition containing mifepristone or a Selective Progesterone Receptor Modulator (SPRM) in a non abortive amount and at least one excipient of a nature and content such that it prevents the intake of tablets by the patient in an abortive amount.

According to another embodiment, the quantity of mifepristone or SPRM is from 1 to 25 mg, preferably from 2 to 10 mg, and more preferably 5 mg.

According to another embodiment, the at least one excipient is a laxative.

According to another embodiment, the at least one excipient is a laxative selected from the group comprising bisacodyl, cascara, docusate, disodic or monosodic phosphate, glycerin, lactulose, magnesium citrate, magnesium hydroxide, methylcellulose, mineral oil, psyllium, matamucil, senna, sorbitol and mixtures thereof.

According to another aspect, the invention is directed to a composition containing a Selective Progesterone Receptor Modulator in a non abortive amount and at least one excipient of a nature and content such that it prevents the intake of the composition by the patient in an abortive amount.

According to another embodiment, the quantity of SPRM is from 1 to 25 mg, preferably from 2 to 10 mg, and more preferably 5 mg.

According to another embodiment, the composition is a tablet.

According to another embodiment, the Selective Progesterone Receptor Modulator is mifepristone.

According to another aspect, the invention is directed to a tablet for oral administration comprising 1 to 25 mg of Selective Progesterone Receptor Modulator, and from 200 to 2500 mg of the at least one excipient.

According to another embodiment, the tablet for oral administration comprises preferably from 5 to 10 mg of Selective Progesterone Receptor Modulator, and from 300 to 1000 mg of the at least one excipient.

According to another embodiment, the Selective Progesterone Receptor Modulator is mifepristone.

According to another embodiment, the tablet for oral administration comprises a swelling agent.

According to another embodiment, the swelling agent is selected from the group consisting of hydroxymethylcellulose, hydroxyethylcellulose, hydroxypropyl methylcellulose having molecular weight from 2,000 to 2,000,000, carboxyvinyl polymers, polyvinyl alcohols, glucans, scleroglucans, chitosans, mannas, galactomannans, xantangums, carrageenans, amylase, alginic acid and salts therof, acrylates, methacrylates, acrylic/methacrylic copolymers, polyanhydrides, polyamino acids, methyl vinyl ethers/maleic anhydride copolymers, carboxymethylcellulose and derivatives thereof, ethylcellulose, methylcellulose and derivatives of cellulose, superporous hydrogels and mixtures thereof.

According to another embodiment, the Selective Progesterone Receptor Modulator can be released according to an immediate, sustained and controlled release.

According to another embodiment, the tablet for oral administration has a dissolution in a 1000 ml USP HCl buffer at pH 1.2, using the paddles method at 75 rpm, of at least 20% at 1 hour, at least 50% at 4 hours and at least 60% at 7 hours, and preferably of at least 30% at 1 hour, 70% at 4 hours and 80% at 7 hours.

According to another embodiment, the tablet for oral administration comprises at least partially swellable hydrophilic polymers in aqueous media, optionally in combination with other adjuvants and further comprising a SPRM, preferably mifepristone, in a single core.

According to another aspect, the invention is directed to a process for the preparation of a tablet according to any one of claims 5 to 12 comprising at least a step of direct compression.

According to another embodiment, the process comprises a step of blending the Selective Progesterone Receptor Modulator, with an excipient having a particle size of less than 60$\mu$m with the Selective Progesterone Receptor Modulator, prior to the compression step.

According to another aspect of the invention, the composition or tablet is for the treatment of gynaecological diseases.

According to another embodiment, the composition or tablet is for the treatment of fibroid tumors.

According to another embodiment, the composition or tablet is for the treatment of leiomyomata.

**BRIEF DESCRIPTION OF THE DRAWINGS:**

Figure 1 represents a photograph of two of the tablets of example 1 at a swollen and non-swollen state.

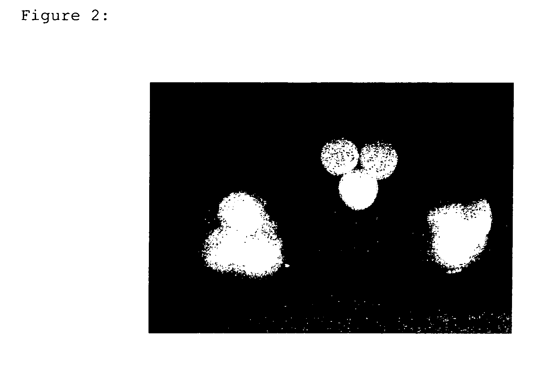

Figure 2 represents a photograph of agglomerated and non agglomerated tablets of example 2.

Figure 3 represents a comparison of the dissolution rate for a single tablet or three tablets of F8, according to example 3.

Figure 4 represents the dissolution profile of tablet F14, according to example 4.

**DETAILED DESCRIPTION OF EMBODIMENTS OF THE INVENTION**

The invention relates to novel galenic formulations of mifepristone or other Selective Progesterone Receptor Modulator (SPRM), used to medically treat uterine fibroid tumors such as leiomyomata.

In one embodiment, the invention relates to a composition or tablet composition containing between 5 and 50 mg of mifepristone or SPRM, especially 2 to 20 mg and preferably 5 mg, together with an excipient preventing misuse of mifepristone.

In a first aspect of the invention, said excipient is a laxative. The type and amount of laxative in the unitary dosage form is such that unpleasant feeling will be observed when e.g. 3 compositions are taken by the patient. Examples of laxative compounds are: bisacodyl sold under the trade name Dulcolax®, cascara, docusate sold under the trade name Colace®, disodic or monosodic phosphate also sold under the trade name fleet phospho-soda®, glycerin, lactulose, magnesium citrate, magnesium hydroxide, methylcellulose sold under the trade name Citrucel®, mineral oil, psyllium, metamucil, senna sold under the trade name Senokot® or sorbitol.

In a second, preferred, aspect of the invention, said excipient provides a GRDF (gastro-retentive delivery form) type composition by swelling (or otherwise expanding) when in contact with the gastric juice present in the stomach. The swelling can notably be such that the swelling ratio (or swelling percentage) is more than 50%, preferably more than 90%, especially more than 200%.

The average residence time of food in the stomach is only about 1 to 3 hours and unless the residence time of the formulation is increased, it will pass to the intestine. In order to prevent the misuse of mifepristone, compositions of the present invention provide dosage forms that expand to sufficient size to delay its passage through the pylorus.

In this embodiment, the core, a first layer surrounding the core or an outer layer contains water-swellable excipient and/or the active ingredient.

The outer layer may further contain a material having bioadhesive properties allowing the positioning and adhesion of pharmaceutical form to the mucosa of the first portion of the gastrointestinal tract.

The layers may also contain gas forming agents, which can optionally be employed to increase the buoyancy. These agents, when in contact with an aqueous media, form a non toxic gas, decrease the density of the pharmaceutical form, and provide floating properties to prolong the gastric residence time into the stomach. Examples of gas forming agents are sodium hydrogen carbonates employed individually or in combination with acids.

In the instant invention, the GRDF is used for its ability to remain in a swollen state in the stomach which prevents the intake of numerous dosage forms, leading necessarily to a fast filling of the stomach. Steric hindrance will thus prevent intake of further dosage forms, and avoid reaching abortive doses of SPRM such as mifepristone.

Preferably, the expansion of the GRDF occurs in less than 15 minutes, preferably less than 10 minutes and more preferably less than 5 minutes.

Also preferably, the swelling causes retention of the pharmaceutical dosage forms in the stomach for three hours or more, more preferably about four hours or more, after which time the drug delivery vehicle either dissolves or degrades into fragments small enough to pass the pylorus.

According to the present invention, the layers may be designed to provide an immediate, sustained or controlled release of the active ingredient into the stomach. Eventhough a rapid delivery of the drug is usually preferred, it may also be advantageous within the scope of the present invention to design layers that provide a prolonged delivery of the drug in order to avoid misusage of the tablets encountered with the intake of several immediate release tablets at a rate of one tablet per minute for example.

Alternatively, a laxative may further be coated to the sustained or controlled release forms, in order to have an immediate release of the laxative together with a prolonged delivery of the SPRM.

A description of GRDF can be found in Expert Opinion, Expert Opin. Drug Deliv. (2006) 3(2):217-233, "Gastroretentive drug delivery systems", by Alexander Streubel, Juergen Siepmann & Roland Bodmeier.

Urquhart and Theeuwes described in patent US 4,434,153 a tablet shaped system consisting of a drug dispersed within a hydrogel-forming polymer matrix which was capable of swelling into the stomach and thus providing a prolonged residence time.

Such systems were improved by Deshpande and colleagues in "Evaluation of films used in development of a novel controlled-release system for gastric retention", Pharm. Res. (1997) 14:815-819, to obtain a controlled-release retention form based on a swellable core comprising the drug and a permeable coating which ensured the integrity of the tablet during the initial swelling phase and controlled the release rate of the drug.

Park and colleagues described enzyme-digestible hydrogels consisting of poly(vinyl pyrrolidone) crosslinked with albumin, which swell to a significant extent and were especially designed for gastroretentive dosage forms. Such hydrogels are also disclosed in Shalaby, WS. and Park, K., "Biochemical mechanical characterization enzyme digestible hydrogels", (1990) Pharmaceutical Research vol. 7, No. 8.

The same group further described a size increasing gastroretentive drug delivery form based on superporous hydrogels capable of swelling to a hundred times of the original size in a few minutes and which are disclosed in Chen J., Park H, Park K., "Synthesis of superporous hydrogels with fast swelling and superabsorbent properties", J. Biomed. Mater. Res. (1999) 44:53-62.

Such GRDF types are also disclosed in patents US 5,780,057; US 6,476,006; EP 1 383 375; EP 339 035; US 5,651,985 and in European Patent Application EP 1 439 826. However, these never related to gastro retentive forms containing a SPRM in a non abortive amount that would avoid its misusage.

According to another aspect of the invention, steric hindrance may also be obtained with tablets that in a non swollen state already have an important size. This way, the evacuation of the tablet via the duodenum as soon as it is swallowed by the patient may be avoided.

An appropriate size may be reached by incorporating an amount of excipients within the range of from 200 to 2500 mg, preferably of from to 300 to 1000 mg and most preferably of about 500 mg.

Without being limitative, tablets according to the present invention may have any of the following structures:

a) a unique core comprising at least partially swellable hydrophilic polymers in aqueous media, optionally in combination with other adjuvants and further comprising the SPRM active ingredient such as mifepristone.

b) a core and eventually a first layer, any of which comprises at least partially swellable hydrophilic polymers in aqueous media, optionally in combination with other adjuvants, at least another layer adjacent to the core or first layer and containing mifepristone or a SPRM in a non abortive amount, comprising generally biodegradable and biocompatible polymeric materials a binder and other adjuvants, whereby the mifepristone or SPRM can be released according to an immediate, sustained and controlled release.

Suitable active ingredients may be any Selective Progesterone Receptor Modulators (SPRM), such as progesterone receptor ligands and encompass broadly any progesterone agonists or antagonists. Suitable active ingredients are those that are useful in the treatment of fibroid tumors and especially leiomyomata. Without being limitative, members of SPRM include asoprisnil (J867) and active compounds of the Progenta®, and the Ulipristal® products such as CDB-4124 PGL-4001 and PGL-2001.

A most preferred SPRM is mifepristone, and especially micronized mifepristone. The d50 of the micronized form is generally less than 30 $\mu$m, preferably less than 20 $\mu$m.

The polymeric substances used to prepare the swellable first layer, are selected from the group consisting of hydroxymethylcellulose, hydroxyethylcellulose, hydroxypropyl methylcellulose having molecular weight from 2,000 to 2,000,000, carboxyvinyl polymers, polyvinyl alcohols, glucans, scleroglucans, chitosans, mannas, galactomannans, xantangums, carrageenans, amylase, alginic acid and salts therof, acrylates, methacrylates, acrylic/methacrylic copolymers, polyanhydrides, polyamino acids, methyl vinyl ethers/maleic anhydride copolymers, carboxymethylcellulose and derivatives thereof, ethylcellulose, methylcellulose and derivatives of cellulose in general, superporous hydrogels in general and mixtures thereof.

Adjuvant includes any of the following components binder, diluent, disintegrating agent, lubricant and anti-static agent and optionally other auxiliary agents, such as surfactants.

In the framework of this invention, the expression "diluent" means any excipient which acts to dilute the formulation without undergoing a chemical reaction with the formulation components. A diluent of the invention includes generally inert carriers or vehicles, be it crystalline or amorphous. Examples of such diluents are derivatives of sugars, such as lactose, saccharose, mannitol, etc., and mixtures thereof. Microcristalline cellulose, its derivatives, and hydrolyzed starch (malto-dextrine) can be used, preferably in low amounts.

In the framework of this invention, the expression "disintegrating agent" means any excipient which exerts an action of disintegrating when the tablet is in an aqueous environment by facilitating the interaction between the components of the layer and the biological fluids, i.e. which exerts an internal pressure leading to breaks in the tablet and ultimately to its disintegration. Examples of disintegrating agents of the invention are cross linked polyvinylpyrrolidone, hydroxypropylcellulose and hydroxypropyl methylcellulose having molecular weight up to 150,000, cross linked sodium carboxymethylcellulose, carboxymethyl starch, sodium carboxymethyl starch, potassium methacrylate-divinylbenzene copolymer, polyvinyl alcohols, amylase, cross linked amylase, sodium starch glycollate starch derivatives, microcrystalline cellulose and cellulose derivatives, alpha, beta, and gamma-cyclodextrin and dextrin derivatives in general, pregelatinized starch and mixtures thereof.

Furthermore, it has been surprisingly found that the composition within the scope of the invention may also have if desired, additional properties that are useful to prevent misusage. Indeed, when several tablets are taken at the same time or at an inappropriate rate, compositions can be chosen so that they stick and agglomerate to each others when gathered in the stomach of the patients. Examples of appropriate excipients that provide sticking properties are any binder such as povidone, sacharrose, or glucose and any soluble diluent such as lactose, sorbitol or mannitol.

Firstly, this has the advantage of significantly lowering the surface of exchange with the outer environment of the agglomerated tablets, and thereby challenges the delivery of the total amount of active ingredient that was absorbed and might be dangerously elevated. In addition, the agglomeration of tablets results in an increased hindrance, having a size that delays its degradation prior to the evacuation through the pylorus. Preventive properties against misusage may then be enhanced if such properties are conferred to the composition of the present invention.

The formulations according to the invention can be prepared conventionally. For example, compositions according to the invention may be prepared by dry blending and formulations may be reached through direct compression or dry granulation techniques.

Due to the fact that, in the embodiment where tablets have an important size at a non swollen state, the SPRM such as mifepristone is present in low amounts, it may be convenient to first blend the active ingredient with part of one or more of the excipients of a similar or close granulometry.

Conventionally, direct compression techniques involve excipients having a size of more than 60 $\mu$m, while the

useful active ingredients can have smaller grades. Micronized mifepristone may for example, consist in 90% of 15 μm particles.

In this eventuality, it becomes convenient to preliminarily blend part of one of more of the excipients provided with a particle size of less than 60μm. Preferably, the excipient is provided with a particle size that is similar or close to the particle size of the active ingredient, within a range that would be considered by the man skilled in the art.

For example, when micronized mifepristone is used, it may be blended with lactose (lactochem microfine®) having a particle size of less than 60 μm, preferably less than 30 μm and more preferably of less than 20 μm.

Should the particle size of the active ingredient be of 60 μm or more, the preliminary blend appears to be less necessary than in the previous situation.

This should ensure a proper homogeneity of the final preparation. In this aspect, lactose appeared to be typically appropriate since it is available in a wide range of powdery grades.

However, granulation (dry, wet or melt) techniques may be particularly useful in the case where the amount of active ingredient is very low, such as less than 1% of the total weight, less than 0.5% or less than 0.1% of active ingredient.

Where a wet granulation technique is applied, the disintegrating excipients may be incorporated at the end of the granulation in order to avoid their contact with water and allow the concentration of the active ingredient.

In order to form a structurally resilient mass upon contact with water or gastric fluid, the blended composition is compacted prior to hydration, by conventional means of dry granulation or direct compression techniques. Mifepristone may be added after comminution of the compacted composition resulting in the mifepristone being extragranular.

As a preferred alternative to dry granulation, the blended composition may be compressed directly into the final pharmaceutical dosage form using direct compression techniques. It produces a more uniform tablet without granules.

The oral dosage forms according to the invention may have a layered construction wherein mifepristone or the SPRM, alone or with other adjuvants, forms an outer layer that is bonded by compression to the other layers formed of the drug delivery vehicle as defined.

According to another embodiment, the SPRM may directly be compressed with the other excipients into a single core by direct compression techniques.

In order to facilitate the production, further adjuvants, suited to direct compression, can be added such as lubricants, anti-static agents or glidants.

In the framework of this invention, the expression "lubricant" means any excipient which ease ejection of the tablet from the tableting dye in which it is formed by compression and improves the flow properties of the composition in powder or granule. Examples of lubricants are talc, magnesium stearate and glyceryl behenate, and mixtures thereof.

In the framework of this invention, the expression "anti-static agent" means any excipient which exerts an anti-static action. Examples of antistatic agents are anhydrous colloidal silica (which may also act as a disintegrant).

In the framework of this invention, the expression "glidant" means any excipient which essentially improves the flow properties of the composition in powder or granule form. An example of glidants is silicon dioxide.

As described, the formulation according to the invention can be prepared by conventional techniques. Therefore, their production on an industrial scale is easily available.

**EXAMPLES**

The following examples illustrate the invention without limiting it.

The following compositions were prepared and formulated in tablet forms by direct compression techniques according to a D12PC format, although different other formats may be appropriate, such as an oblong format (16x10 mm). All of the forms have a total weight of 490 mg, contain 5 mg of mifepristone, and the composition is expressed in percentage of the total weight of the composition.

|  | Matrix Nol | F8 (% weight) | F14 (% weight) |
|---|---|---|---|
| croscarmellose sodium |  | 10 | 15 |
| crospovidone | 10 |  |  |
| microcrystallinecellulose (MCC) | 62.98 | 46.98 | 54.98 |
| lactose |  | 16 | 5 |
| active ingredient (micronized mifepristone) | 1.02 | 1.02 | 1.02 |
| kollidon VA 64 | 10 | 10 | 10 |
| hydroxypropylmethyl cellulose (HPMC 100.000®) | 14 | 14 | 12 |

(continued)

|  | Matrix Nol | F8 (% weight) | F14 (% weight) |
|---|---|---|---|
|  |  |  |  |
| magnesium stearate | 2 | 2 | 2 |
|  |  |  |  |
| swelling ratio | 210% | 95% | 246% |
| Sticking (0->4) | 1 | 4 | 1 |

Example 1: Study of the swelling properties for the prepared forms.

The following test, evaluated the swelling properties of the compositions according to the present invention into the stomach once swallowed.

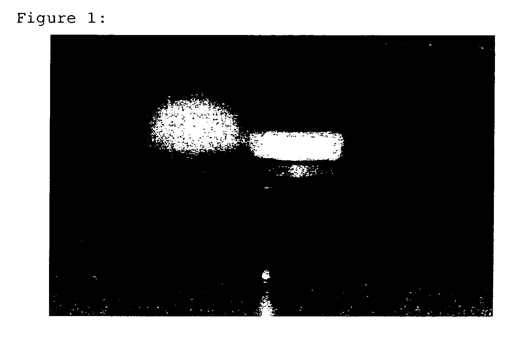

The test was performed using a dissolution bath of 1000 ml, at 37°C and pH = 1,2 which correspond to the acidity of the gastric juice. The bath was provided with a paddle rotating agitator (75 rpm). Each of 6 formulated tablets was tested during 10 minutes, after which the final volume (Vf) was compared to the initial non swollen state (Vi). Volume was evaluated from the diameter and height of the tablet. Figure 1 represents a picture of a tablet according to the invention respectively at a swollen and non swollen state. The presented swelling ratios, should however, be considered qualitatively, due to the difficulty of accurately measuring the volume of the tablets at a swollen state. The swelling percentage is given using the formula:

$$\frac{(Vf - Vi)}{Vi}\ (\%).$$

Example 2: Sticking properties of the prepared forms.

A Similar bath system as in the prior test was used to evaluate the sticking and agglomeration of the compositions according to the invention.

In similar conditions of temperature and acidity, three tablets were inserted in a bath during 10 to 15 minutes. The efficacy of the sticking of the resulting form was noted between 0 and 4 from the visual aspect of the agglomerate. 0 corresponds to a non agglomerated state and 4 to a homogenous single form.

Example 3: Dissolution of the F8 tablets.

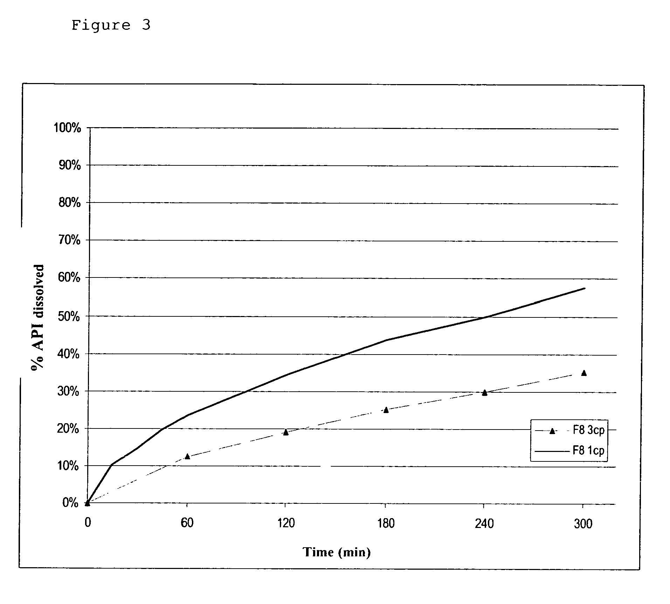

A similar bath system as in the prior tests was used to evaluate the rate of dissolution of tablets F8. At 37°C, and pH = 1.2, under stirring (75 rpm) either one tablet or three tablets were introduced in a basket into the bath. The obtained dissolution profile is the one of figure 3, which clearly shows that when several of these tablets are gathered into the stomach, they show a lower rate of dissolution than for a single tablet.

Example 4: Dissolution of the F14 tablets.

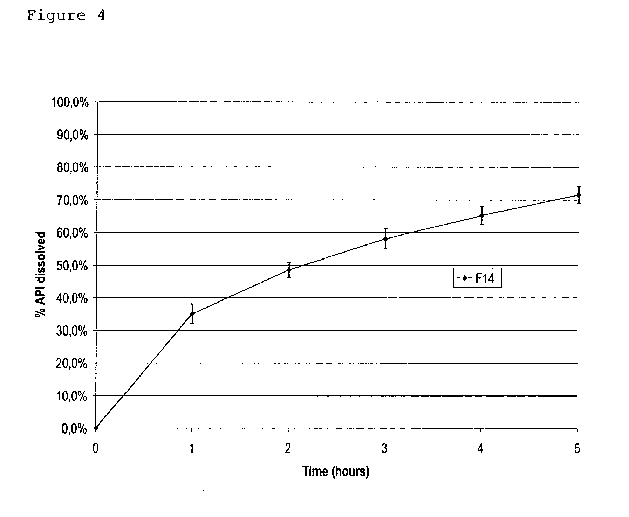

A similar bath system as in the prior tests was used to evaluate the rate of dissolution of tablets F14. At 37°C, and pH = 1.2, under stirring (75 rpm) each one of 6 tablets was introduced in a basket into a bath. The obtained dissolution profile is the one of figure 4. In the present example, the tablet appeared to float into the bath which implies that the dissolution profile should be more rapid when using a sinker. The present tablet illustrates a particularly appropriate profile which allows the rapid delivery of the drug to the patient without encountering the previously presented misusage, especially by repetitive intakes of tablets. This formulation provides an advantageous dissolution of at least 20% at 1 hour, at least 50% at 4 hours and at least 60% at 7 hours, and more particularly of at least 30% at 1 hour, 70% at 4 hours and 80% at 7 hours.

**Claims**

1. A composition containing a Selective Progesterone Receptor Modulator in a non abortive amount and at least one excipient of a nature and content such that it prevents the intake of the composition by the patient in an abortive amount.

2. The composition according to claim 1, wherein the quantity of SPRM is from 1 to 25 mg, preferably from 2 to 10 mg, and more preferably 5 mg.

3. The composition according to claim 1 or 2, which is a tablet.

4. The composition according to anyone of claims 1 to 3, wherein the Selective Progesterone Receptor Modulator is mifepristone.

5. A tablet for oral administration comprising 1 to 25 mg of Selective Progesterone Receptor Modulator, and from 200 to 2500 mg of at least one excipient.

6. The tablet according to claim 5 comprising preferably from 5 to 10 mg of Selective Progesterone Receptor Modulator, and from 300 to 1000 mg of the at least one excipient.

7. The tablet according to claim 5 or 6, wherein the Selective Progesterone Receptor Modulator is mifepristone.

8. The tablet according to any of claims 5 to 7, comprising a swelling agent.

9. The tablet according to claim 8, wherein the swelling agent is selected from the group consisting of hydroxymethylcellulose, hydroxyethylcellulose, hydroxypropyl methylcellulose having molecular weight from 2,000 to 2,000,000, carboxyvinyl polymers, polyvinyl alcohols, glucans, scleroglucans, chitosans, mannas, galactomannans, xantangums, carrageenans, amylase, alginic acid and salts therof, acrylates, methacrylates, acrylic/methacrylic copolymers, polyanhydrides, polyamino acids, methyl vinyl ethers/maleic anhydride copolymers, carboxymethylcellulose and derivatives thereof, ethylcellulose, methylcellulose and derivatives of cellulose, superporous hydrogels and mixtures thereof.

10. The tablet according to any of claims 5 to 9, whereby the Selective Progesterone Receptor Modulator can be released according to an immediate, sustained and controlled release.

11. The tablet according to any one of claim 5 to 10 having a dissolution in a 1000 ml USP HCl buffer at pH 1.2, using the paddles method at 75 rpm, of at least 20% at 1 hour, at least 50% at 4 hours and at least 60% at 7 hours, and preferably of at least 30% at 1 hour, 70% at 4 hours and 80% at 7 hours.

12. The tablet according to any one of claim 5 to 11, comprising at least partially swellable hydrophilic polymers in aqueous media, optionally in combination with other adjuvants and further comprising a SPRM, preferably mifepristone, in a single core.

13. The composition or tablet according to any one of claims 1 to 12, which is a GRDF.

14. A process for the preparation of a tablet according to any one of claims 5 to 13 comprising at least a step of direct compression.

15. The process according to claim 14, comprising a step of blending the Selective Progesterone Receptor Modulator, with an excipient having a particle size of less than 60 $\mu$m with said Selective Progesterone Receptor Modulator, prior to the compression step.

16. The composition or tablet according to any one of claims 1 to 13 for the treatment of gynaecological diseases.

17. The composition or tablet according to any one of claims 1 to 13 for the treatment of fibroid tumors.

18. The composition or tablet according to any one of claims 1 to 13 for the treatment of leiomyomata.

Figure 2:

Figure 3

Figure 4

Europäisches
Patentamt

European
Patent Office

Office européen
des brevets

# EUROPEAN SEARCH REPORT

**Application Number**

EP 09 29 0034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/031491 A1 (H.L.LEVINE ET AL.) 8 February 2007 (2007-02-08) * claims * * examples * * figure 1 * | 1-16 | INV. A61K9/00 A61K9/20 A61K31/567 |
| A | EP 1 987 814 A (EXELGYN) 5 November 2008 (2008-11-05) * claims * * examples * | 1-18 | |
| A | WO 2007/103510 A (DANCO) 13 September 2007 (2007-09-13) * claims * | 1-18 | |
| A | US 2005/220715 A1 (T. LIN) 6 October 2005 (2005-10-06) * claims * * examples * | 1-18 | |
| | | | TECHNICAL FIELDS SEARCHED (IPC) |
| | | | A61K |

The present search report has been drawn up for all claims

| Place of search | Date of completion of the search | Examiner |
|---|---|---|
| The Hague | 19 May 2009 | Scarponi, Ugo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 29 0034

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2009

| Patent document cited in search report | | Publication date | Patent family member(s) | | Publication date |
|---|---|---|---|---|---|
| US 2007031491 | A1 | 08-02-2007 | NONE | | |
| EP 1987814 | A | 05-11-2008 | EP | 1990044 A1 | 12-11-2008 |
| WO 2007103510 | A | 13-09-2007 | NONE | | |
| US 2005220715 | A1 | 06-10-2005 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CA 2029940
- US 4434153 A
- US 5780057 A
- US 6476006 B
- EP 1383375 A
- EP 339035 A
- US 5651985 A
- EP 1439826 A

### Non-patent literature cited in the description

- **Murphy, A.A et al.** Regression of Uterine Leiomyomata in Response to the Antiprogesterone RU 486. *Journal of Clinical Endocrinology and Metabolism,* 1993, vol. 76 (2
- *Expert Opinion, Expert Opin. Drug Deliv.,* 2006, vol. 3 (2), 217-233
- Evaluation of films used in development of a novel controlled-release system for gastric retention. *Pharm. Res.,* 1997, vol. 14, 815-819
- **Shalaby, WS. ; Park, K.** Biochemical mechanical characterization enzyme digestible hydrogels. *Pharmaceutical Research,* 1990, vol. 7 (8
- **Chen J. ; Park H ; Park K.** Synthesis of superporous hydrogels with fast swelling and superabsorbent properties. *J. Biomed. Mater. Res.,* 1999, vol. 44, 53-62